(19) Europäisches Patentamt
European Patent Office
Office européen des brevets

(11) Veröffentlichungsnummer: **0 075 165**
**B1**

(12) # EUROPÄISCHE PATENTSCHRIFT

(45) Veröffentlichungstag der Patentschrift: **29.10.86**

(51) Int. Cl.⁴: **C 07 D 471/04**, A 61 K 31/505 // (C07D471/04, 239:00, 221:00)

(21) Anmeldenummer: **82108170.0**

(22) Anmeldetag: **04.09.82**

(54) **9,10-Substituierte 2-Mesitylimino-3-alkyl-3,4,6,7-tetrahydro-2H-pyrimido(6,1-a)isochinolin-4-one, Verfahren zu ihrer Herstellung und ihre Verwendung als Arzneimittel.**

(30) Priorität: **10.09.81 DE 3135831**

(43) Veröffentlichungstag der Anmeldung: **30.03.83 Patentblatt 83/13**

(45) Bekanntmachung des Hinweises auf die Patenterteilung: **29.10.86 Patentblatt 86/44**

(84) Benannte Vertragsstaaten: **AT BE CH DE FR GB IT LI NL SE**

(56) Entgegenhaltungen:
**EP - A - 0 010 759**
**GB - A - 1 597 717**
**US - A - 4 013 663**

**Chemical Abstracts Band 95, Nr. 9, 31 August 1981, Columbus, Ohio, USA B.LAL et al. "Cardiovascular and antihypertensive activity of the new vasodilator 9,10-dimethoxy-2-mesity-limino-3-methyl-3,4,6,7-tetra-hydro-2H-pyrimido (6,1-a) isoquinolin-4-one hydrochloride (HL 725) Seite 73, Abstract Nr. 73469f**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Lal, Bansi, Dr., Advani Apartments 30 Mulund (West), Bombay-400 080 (IN)**
Erfinder: **Dohadwalla, Alihussein Nomanbhai, Dr., Noman Mansion 139C Cumballa Hill, Bombay-400 036 (IN)**
Erfinder: **Aroskar, Vijay Atmaram Asha Apartments 5, Linking Road Extension 17/C, Santa Cruz Bombay-400 054 (IN)**
Erfinder: **Dadkar, Nandkumar Keshav, Dr. Flat No. 8, CII, Municipal Quaters 358 Khan Abdul Gaffar Rd. Worli, Bombay-400 018 (IN)**
Erfinder: **Dornauer, Horst, Dr., Am Kirchplatz 14, D-6233 Kelkheim (Taunus) (DE)**
Erfinder: ***Mascarenhas, Julius, Matharpacady 16A Mazagon, Bombay-400 010 (IN)***
Erfinder: **de Souza, Noel John, Dr., Avanti Central Avenue 702, Santa Cruz Bombay-400 054 (IN)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft
9,10-substituierte 2-Mesitylimino-3-alkyl-3,4,6,7-tetrahydro-2H-pyrimido(6,1-a)isochinolin-4-one
und ihre pharmazeutisch verträglichen Salze, ihre Herstellung und ihre Verwendung als Arzneimittel, insbesondere als blutdrucksenkende Mittel, sowie diese Verbindungen enthaltende pharmazeutische Zubereitungen.

Die erfindungsgemässen Verbindungen sind substituierte
9-/10-Alkoxy/(3′-substituierte Amino-2′-OR[1]-propoxy)-2-mesitylimino-3-alkyl-3,4,6,7-tetrahydro-2H-pyrimido(6,1-a)-iso-chinolin-4-one
der Formel I

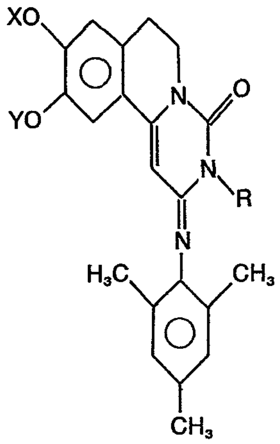

I

in welcher R geradkettiges oder verzweigtes $C_1$–$C_4$-Alkyl und einer der Reste X und Y geradkettiges oder verzweigtes $C_1$–$C_4$-Alkyl, der andere $-CH_2CH(OR^1)CH_2NR^2R^3$ bedeuten, wobei $R^1$, $R^2$ und $R^3$ gleich oder verschieden sind und Wasserstoff oder geradkettiges oder verzweigtes $C_1$–$C_4$-Alkyl bedeuten oder $R^2$ und $R^3$ zusammen mit dem Stickstoffatom, an das sie gebunden sind, einen Piperazino-, Morpholino- oder Piperidino-Rest darstellen, welche unsubstituiert oder substituiert sind mit $C_1$–$C_4$-Alkyl, $C_2$–$C_5$-Carbalkoxy, Pyridyl, Naphthyl, Phenyl oder 1- bis 3-fach mit Halogen, Hydroxy, Methyl, Ethyl, Methoxy, Nitro oder Trifluormethyl substituiertem Phenyl, sowie deren pharmazeutisch verträgliche Salze.

Geeignete $C_1$–$C_4$-Alkylgruppen für X, Y, $R^1$, $R^2$, $R^3$ sind Methyl, Äthyl, n-Propyl, Isopropyl, Isobutyl oder tert.-Butyl.

Als geeignete Salze der erfindungsgemässen
9-/10-Alkoxy/(3′-subst. Amino-2′-OR[1]-propoxy)-2-mesitylimino-3-alkyl-3,4,6,7-tetrahydro-2H-pyrimido(6,1-a)isochinolin-4-one
seien beispielsweise genannt Salze mit anorganischen oder organischen Säuren, wie z.B. Hydrochloride, Hydrobromide, Hydrojodide, Sulfate, Phosphate, Acetate, Propionate, Oxalate, Tartrate, Zitrate, Maleate, Fumarate, Isethionate, Succinate, Pamoate, Pivalate usw.

Bevorzugte Substituenten sind: Methyl oder Äthyl für R, geradkettiges oder verzweigtes $C_1$–$C_4$-Alkyl für einen der beiden Reste X und Y, und $-CH_2CH(OH)CH_2R^2R^3$ für den anderen der Reste X und Y, worin $R^2$ Wasserstoff und $R^3$ geradkettiges oder verzweigtes $C_1$–$C_4$-Alkyl bedeutet oder $R^2$ und $R^3$ zusammen mit dem Stickstoffatom, an das sie gebunden sind, einen der unsubstituierten oder wie oben angegebenen substituierten Piperazino-, Morpholino- oder Piperidino-Reste darstellen.

Besonders bevorzugte Verbindungen gemäss der Erfindung sind:
9-Methoxy-10-[3′-(4-phenylpiperazino)-2′-hydroxypropoxy]-2-mesitylimino-3-methyl-3,4,6,7-tetrahydro-2H-pyrimido-(6,1-a)isochinolin-4-on-dihydrochlorid-dihydrat.
9-Methoxy-10-[3′-(4-(2-pyridyl)piperazino)-2-hydroxypropoxy]-2-mesitylimino-3-methyl-3,4,6,7-tetrahydro-2H-pyrimido-(6,1-a)isochinolin-4-on-trihydrochlorid-dihydrat.
9-Methoxy-10-[3′-(4-phenylpiperidino)-2′-hydroxypropoxy]-2-mesitylimino-3-methyl-3,4,6,7-tetrahydro-2H-pyrimido-(6,1-a)isochinolin-4-on.
10-Methoxy-9-[3′-(4-phenylpiperidino)-2′-hydroxypropoxy]-2-mesitylimino-3-methyl-3,4,6,7-tetrahydro-2H-pyrimido-(6,1-a)isochinolin-4-on-dihydrochlorid-sesquihydrat.

In der folgenden Tabelle sind einige der neuen substituierten
9-/10-Alkoxy/(3′-subst. amino-2′-OR[1]-propoxy)-2-mesitylimino-3-alkyl-3,4,6,7-tetrahydro-2H-pyrimido(6,1-a)isochinolin-4-one
aufgeführt.

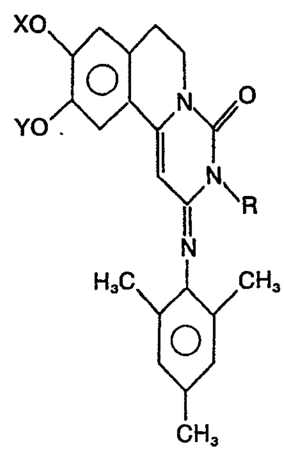

I

| X | Y | Salz | Fp. (°C) |
|---|---|---|---|
| $CH_3$ | $CH_2CHOHCH_2NHCHMe_2$ | 2 HCl | 246–248 |
| $CH_3$ | $CH_2CHOHCH_2NHCMe_3$ | 2 HCl | 237–239 |
| $CH_3$ | $CH_2CHOHCH_2{-}NHCH_2CHMe_2$ | 2 HCl · $H_2O$ | 228–229 |

Die vorliegende Erfindung betrifft ferner ein Verfahren zur Herstellung der 9-/10-Alkoxy/(3′-subst.-amino-2′-OR[1]-propoxy)-2-mesitylimino-3-alkyl-3,4,6,7-tetrahydro-2H-pyrimido(6,1-a)isochinolin-4-one der Formel I und ihrer pharmazeutisch verträglichen Salze, dadurch gekennzeichnet, dass man eine Verbindung der Formel II

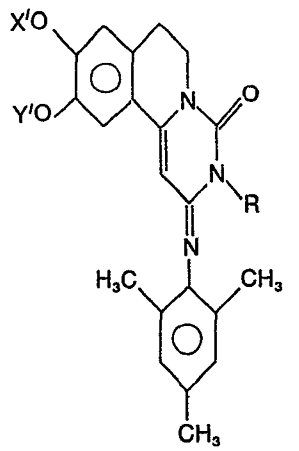

II

worin einer der Reste X′ und Y′ Wasserstoff und der andere geradkettiges oder verzweigtes $C_1$–$C_4$-Alkyl bedeutet, mit einem Epihalogenhydrin der Formel III

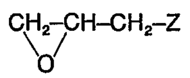

III

worin Z ein Halogenatom, wie z.B. Brom, bedeutet, in Gegenwart einer Base, wie z.B. Kaliumcarbonat, Natriumhydrid, und eines Lösungsmittels wie Aceton, Dimethylformamid, Dimethylsulfoxid, halogenierte Kohlenwasserstoffe, z.B. Chloroform, reagieren lässt, wobei die Reaktion durch Anwendung von Hitze, z.B. bei Siedetemperatur des Lösungsmittels, beschleunigt oder beendet wird, und man das Produkt mit einer Verbindung der Formel IV

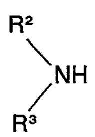

IV

worin $R^2$ und $R^3$ die vorgenannten Bedeutungen haben, behandelt, um so eine erfindungsgemässe Verbindung der Formel I zu erhalten.

Die Verbindungen der Formel II werden zweckmässigerweise aus den entsprechenden 9,10-Dimethoxyderivaten (s. Deutsche Offenlegungsschrift Nr. 2 720 085) durch übliche Demethylierungsverfahren, z.B. Behandeln mit Jod- oder Bromwasserstoffsäure, Pyridinhydrochlorid oder Natriumäthylmercaptid/Dimethylformamid hergestellt.

Die Verbindungen gemäss der vorliegenden Erfindung umfassen auch die pharmazeutisch verträglichen Salze der Verbindungen der Formel I. Diese Salze werden durch Behandlung der Verbindungen der Formel I mit einer entsprechenden Menge einer geeigneten Säure, normalerweise in einem Lösungsmittel, hergestellt. Beispiele solcher brauchbaren anorganischen und organischen Säuren sind oben bereits aufgeführt.

Die erfindungsgemässen Verbindungen besitzen wertvolle pharmakologische Eigenschaften, z.B. blutdrucksenkende Wirkung, wie Versuche an Ratten mit spontanem Bluthochdruck (SH-Ratten) gezeigt haben. Es wird angenommen, dass diese blutdrucksenkende Wirkung das Ergebnis peripherer Gefässerweiterung ist, jedenfalls zum Teil.

Strukturell ähnliche Verbindungen mit blutdrucksenkender Wirkung sind aus den Druckschriften GB-A-1 597 717 und C.A. 95, Nr. 9 (1981) 73 469 bekannt. Der Substituent $-OCH_2-CH(OH)CH_2NHR_4$ in Verbindungen mit Betablokker-Wirkung ist ferner aus US-A-4 013 663 bekannt.

Die Fähigkeit der erfindungsgemässen Verbindungen, den Blutdruck in SH-Ratten zu senken, zeigt, dass diese Verbindungen und ihre Salze für die Behandlung von Bluthochdruck in der Human- und Veterinärmedizin geeignet sind. Die erfindungsgemässen Verbindungen können auch in Kombination mit anderen pharmakologisch wirksamen Mitteln, z.B. Diuretika, herzgefässerweiternde Mittel und Lipidsenker, angewandt werden.

Die erfindungsgemässen Wirkstoffe können peroral, parenteral (intramuskulär, intravenös, subkutan), rektal oder lokal äusserlich, ggf. in Form von Sprays, verabreicht werden.

Folgende Dosierungen zur Senkung des Blutdrucks werden bei Säugetieren angewandt: Tagesdosis von 0.1 bis 200 mg, Dosiseinheit 0.1 bis 25 mg.

Die neuen Verbindungen können entweder allein oder in Mischung mit pharmakologisch verträglichen Trägermaterialien angewandt werden. Für eine orale Verabreichung werden die Wirkstoffe mit üblichen Substanzen vermischt und in eine gebräuchliche Verabreichungsform, z.B. Tabletten, Steckkapseln, wässrig/alkoholische oder ölige Suspensionen oder Lösungen, gebracht. Geeignete inerte Trägermaterialien sind z.B. Magnesiumcarbonat, Milchzucker oder Maisstärke, die mit anderen Substanzen wie Magnesiumstearat versetzt sein können. Die Zubereitungen können in Form von trockenem oder feuchtem Granulat hergestellt werden. Als ölige Träger oder Lösungsmittel können pflanzliche oder tierische Öle, wie Sonnenblumenöl oder Lebertran, eingesetzt werden.

In Notfällen können die Wirkstoffe intravenös injiziert werden. Zu diesem Zweck werden die Wirkstoffe oder ihre physiologisch verträglichen Salze, sofern sie ausreichend löslich sind, in üblichen Hilfsstoffen, die auch als Lösungsvermittler oder Puffer dienen können, gelöst.

Physiologisch verträgliche Salze werden z.B. mit folgenden Säuren gebildet: Salzsäure, Brom- und Jodwasserstoffsäure, Phosphorsäure, Schwefelsäure, Methylschwefelsäure, Amidosulfonsäure, Salpetersäure, Weinsäure, Milchsäure, Malonsäure, Fumarsäure, Oxalsäure, Zitronensäure, Äpfelsäure, Schleimsäure, Benzoesäure, Salicylsäure, Acetursäure, Embonsäure, Naphthalin-1,5-disulfonsäure, Ascorbinsäure, Phenylessigsäure, p-Aminosalicylsäure, Hydroxyäthylsulfonsäure, Benzolsulfonsäure oder Kunstharze, die saure Gruppen enthalten, z.B. die mit Ionenaustauscherwirkung.

Geeignete Lösungsmittel für die intavenöse Verabreichung sind z.B. Wasser, physiologische Kochsalzlösung oder verdünnte Alkohole wie Äthanol, Propandiol oder Glyzerin, ferner Zuckerlösungen wie Glucose- oder Mannitlösungen, es kommt aber auch eine Mischung der genannten Lösungsmittel infrage.

Die folgenden Beispiele sollen die Erfindung näher erläutern:

Beispiel 1

9-Methoxy-10-[3′-(4-phenylpiperazino)-2′-hydroxypropoxy]-2-mesitylimino-3-methyl-3,4,6,7-tetrahydro-2H-pyrimido(6,1-a)isochinolin-4-on-dihydrochlorid-dihydrat

Stufe a)

Zu einer Suspension von wasserfreiem Kaliumcarbonat (18.0 g) in trockenem Aceton (400 ml) wurde
9-Methoxy-10-hydroxy-2-mesitylimino-3-methyl-3,4,6,7-tetrahydro-2H-pyrimido(6,1-a)-isochinolin-4-on- (5.67 g)
und Epibromhydrin (3.03 g) gegeben, und die Mischung wurde 22 Stunden am Rückfluss gekocht. Die Reaktionsmischung wurde dann gefiltert. Die Verdampfung des Filtrats im Vakuum ergab einen Feststoff, der aus Methanol kristallisiert wurde. Ausbeute 4,71 g, Fp. 171–172 °C.

Stufe b)

Eine Mischung des in Stufe a) erhaltenen Produkts (3.22 g) in Chloroform (125 ml) wurde mit N-Phenylpiperazin (7.2 g) behandelt und 30 Stunden am Rückfluss gekocht. Überschüssiges Chloroform wurde im Vakuum abdestilliert. Der Rückstand wurde an einer Kieselgel-Kolonne unter Benutzung von Benzol/Chloroform (1:1) als Laufmittel chromatographiert.

Die Verdampfung der Fraktionen die das gewünschte Produkt enthalten ergab einen Feststoff, der durch Behandlung mit ätherischer Salzsäure in Äthanol in sein Hydrochlorid übergeführt wurde. Das Dihydrochlorid-Dihydrat wurde aus Äthanol/Äther kristallisiert. Ausbeute 2.9 g, Fp. 208–210 °C.

Beispiel 2

9-Methoxy-10-[3′-(4-(2-pyridyl)piperazino)-2′-hydroxy-propoxy]-2-mesitylimino-3-methyl-3,4,6,7-tetrahydro-2H-pyrimido(6,1-a)isochinolin-4-on-trihydrochlorid-dihydrat

Stufe a)

Stufe a) von Beispiel 1 wurde wiederholt.

Stube b)

Das Verfahren aus Stufe b) von Beispiel 1 wurde wiederholt mit N-(2-Pyridyl)-piperzin anstelle von N-Phenylpiperazin, wobei das Trihydrochlorid erhalten wurde. Ausbeute 74%, Fp. 195–197 °C.

Beispiel 3

9-Methoxy-10-[3′-(4-phenylpiperidino)-2′-hydroxypropoxy]-2-mesitylimino-3-methyl-3,4,6,7-tetrahydro-2H-pyrimido-(6,1-a)isochinolin-4-on-dihydrochlorid-monohydrat

Stufe a)

Stufe a) von Beispiel 1 wurde wiederholt.

Stufe b)

Das Verfahren aus Stufe b) von Beispiel 1 wurde wiederholt mit 4-Phenylpiperidin anstelle von N-Phenylpiperazin, wobei das Dihydrochlorid-Monohydrat erhalten wurde. Ausbeute: 57%, Fp. 225–229 °C.

Beispiel 4

10-Methoxy-9-[3′-(4-phenylpiperidino)-2′-hydroxy-propoxy]-2-mesitylimino-3-methyl-3,4,6,7-tetrahydro-2H-pyrimido(6,1-a)isochinolin-4-on-dihydrochlorid-sesquihydrat

Das Verfahren aus Stufen a) und b) von Beispiel 1 wurde wiederholt, wobei
9-Hydroxy-10-methoxy-2-mesitylimino-3-methyl-3,4,6,7-tetrahydro-2H-pyrimido(6,1-a)isochinolin-4-on
anstelle von
9-Methoxy-10-hydroxy-2-mesitylimino-3-methyl-3,4,6,7-tetrahydro-2H-pyrimido(6,1-a)isochinolin-4-on
in Stufe a) und 4-Phenylpiperidin anstelle von N-Phenylpiperazin in Stufe b) eingesetzt wurden. Es wurde das Dihydrochlorid-Sesquihydrat erhalten. Ausbeute: 60%, Fp. 171–173 °C.

**Patentansprüche für die Vertragsstaaten BE, CH, DE, FR, GB, IT, LI, NL, SE**

1. Verbindungen der Formel I

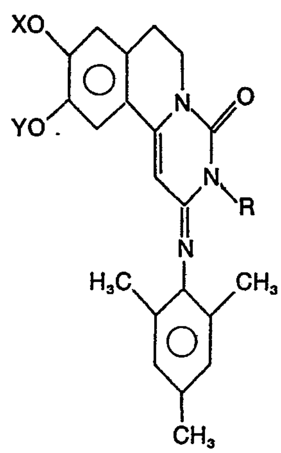

I

worin

R $C_1$–$C_4$-Alkyl bedeutet,
einer der beiden Substituenten X und Y $C_1$–$C_4$-Alkyl und der andere einen Rest der Formel $-CH_2CH(OR^1)CH_2NR^2R^3$ darstellt, worin $R^1$, $R^2$ und $R^3$ gleich oder verschieden sind und Wasserstoff oder $C_1$–$C_4$-Alkyl bedeuten, oder $R^2$ und $R^3$ zusam-

men mit dem Stickstoffatom, an das sie gebunden sind den Piperazino-, Morpholino- oder Piperidino-Rest darstellen, welche unsubstituiert oder substituiert sind mit $C_1$–$C_4$-Alkyl, $C_2$–$C_5$-Carbalkoxy, Pyridyl, Naphthyl, Phenyl oder 1- bis 3-fach mit Halogen, Hydroxy, Methyl, Äthyl, Methoxy, Nitro oder Trifluormethyl substituiertem Phenyl, sowie deren pharmazeutisch verträgliche Salze.

2. Verbindungen der Formel I nach Anspruch 1, worin R Methyl bedeutet und einer der Substituenten X und Y $C_1$–$C_4$-Alkyl und der andere einen Rest der Formel $-CH_2CH(OR^1)CH_2NR^2R^3$ darstellt, worin $R^1$ und $R^2$ Wasserstoff und $R^3$ $C_1$–$C_4$-Alkyl bedeuten oder $R^2$ und $R^3$ zusammen mit dem Stickstoffatom, an das sie gebunden sind, den Piperazino- Morpholino- oder Piperidino-Rest darstellen, welche unsubstituiert oder substituiert sind mit $C_1$–$C_4$-Alkyl, $C_2$–$C_5$-Carbalkoxy, Pyridyl, Naphthyl, Phenyl oder 1- bis 3-fach mit Halogen, Hydroxy, Methyl, Äthyl, Methoxy, Nitro oder Trifluormethyl substituiertem Phenyl, sowie deren pharmazeutisch verträgliche Salze.

3. Verfahren zur Herstellung von Verbindungen der Formel I

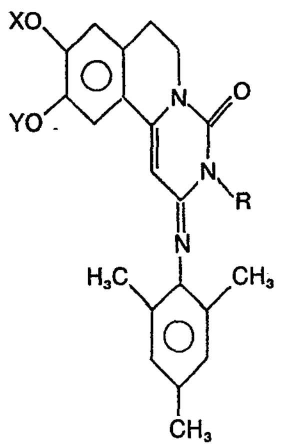

I

dadurch gekennzeichnet, dass eine Verbindung der Formel II

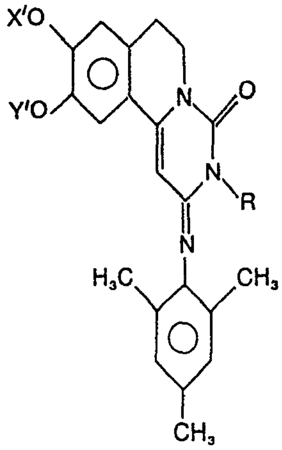

II

worin einer der beiden Substituenten X' und Y' Wasserstoff und der andere $C_1$–$C_4$-Alkyl bedeutet, mit einem Epihalohydrin der Formel III

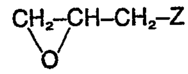

III

worin Z ein Halogenatom bedeutet, umgesetzt wird und das erhaltene Produkt mit einer Verbindung der Formel IV

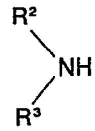

IV

worin $R^2$ und $R^3$ die obengenannten Bedeutungen haben, umgesetzt wird.

4. Arzneimittel, gekennzeichnet durch einen Gehalt an einer Verbindung der in Anspruch 1 genannten Formel I.

5. Verbindung der Formel I gemäss Anspruch 1 oder ein pharmazeutisch verträgliches Salz zur Verwendung als Arzneimittel.

**Patentansprüche für den Vertragsstaat AT**

1. Verfahren zur Herstellung von Verbindungen der Formel I

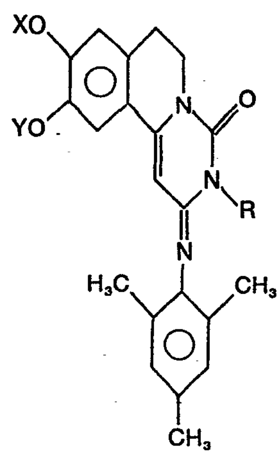

I

worin

R $C_1$–$C_4$-Alkyl bedeutet,

einer der beiden Substituenten X und Y $C_1$–$C_4$-Alkyl und der andere einen Rest der Formel $-CH_2CH(OR^1)CH_2NR^2R^3$ darstellt, worin $R^1$, $R^2$ und $R^3$ gleich oder verschieden sind und Wasserstoff oder $C_1$–$C_4$-Alkyl bedeuten, oder $R^2$ und $R^3$ zusammen mit dem Stickstoffatom, an das sie gebunden sind den Piperazino- Morpholino- oder Piperidino-Rest darstellen, welche unsubstituiert oder substituiert sind mit $C_1$–$C_4$-Alkyl, $C_2$–$C_5$-Carbalkoxy, Pyridyl, Naphthyl, Phenyl oder 1- bis 3-fach mit Halogen, Hydroxy, Methyl, Äthyl, Methoxy, Nitro oder Trifluormethyl substituiertem Phenyl, sowie deren pharmazeutisch verträgliche Salze,

dadurch gekennzeichnet, dass eine Verbindung der Formel II

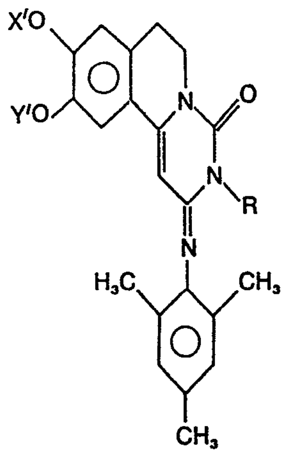

II

worin einer der beiden Substituenten X′ und Y′ Wasserstoff und der andere $C_1$–$C_4$-Alkyl bedeutet, mit einem Epihalohydrin der Formel III

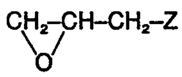

III

worin Z ein Halogenatom bedeutet, umgesetzt wird und das erhaltene Produkt mit einer Verbindung der Formel IV

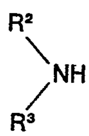

IV

worin $R^2$ und $R^3$ die obengenannten Bedeutungen haben, umgesetzt wird, und gegebenenfalls die erhaltene Verbindung der Formel I in ein pharmazeutisch verträgliches Salz übergeführt wird.

2. Verfahren nach Anspruch 1, dadurch gekennzeichnet, dass eine Verbindung der Formel II, worin R Methyl bedeutet und einer der Substituenten X′ und Y′ $C_1$–$C_4$-Alkyl und der andere Wasserstoff bedeutet, zunächst mit einer Verbindung der Formel III umgesetzt und das erhaltene Produkt dann mit einer Verbindung der Formel IV, worin $R^2$ Wasserstoff und $R^3$ $C_1$–$C_4$-Alkyl bedeuten oder $R^2$ und $R^3$ zusammen mit dem Stickstoffatom, an das sie gebunden sind, den Piperazino, Piperidino oder Morpholino-Rest darstellen, welche unsubstituiert sind oder substituiert sind mit $C_1$–$C_4$-Alkyl, $C_2$–$C_5$-Carbalkoxy, Pyridyl, Naphthyl, Phenyl oder 1- bis 3-fach mit Halogen, Hydroxy, Methyl, Äthyl, Methoxy, Nitro oder Trifluormethyl substituiertem Phenyl, umgesetzt wird.

**Claims for the contracting States: BE, CH, DE, FR, GB, IT, LI, NL, SE**

1. A compound of the formula I

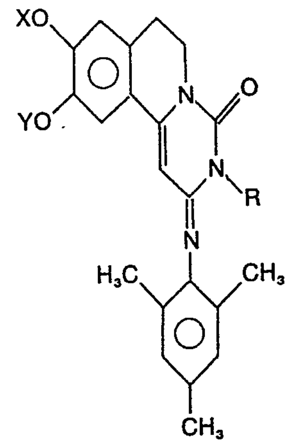

I

wherein R denotes $C_1$–$C_4$-alkyl, one of the two substituents X and Y is $C_1$–$C_4$-alkyl and the other is a radical of the formula $-CH_2CH(OR^1)CH_2NR^2R^3$ $R^1$, $R^2$ and $R^3$ being identical or different and denoting hydrogen or $C_1$–$C_4$-alkyl, or $R^2$ and $R^3$, together with the nitrogen atom to which they are bonded, denoting the piperazino, piperidino or morpholino-radical, which are unsubstituted or are substituted by $C_1$–$C_4$-alkyl, $C_2$–$C_5$-carboalkoxy, pyridyl, naphthyl, phenyl or phenyl, which is 1 to 3 times substituted with halogen, hydroxy, methyl, ethyl, methoxy, nitro or trifluoromethyl, and a pharmaceutically tolerated salt thereof.

2. A compound of the formula I as claimed in claim 1, wherein R denotes methyl and one of the substituents X and Y is $C_1$–$C_4$-alkyl and the other is a radical of the formula $-CH_2CH(OR^1)CH_2NR^2R^3$, $R^1$ and $R^2$ denoting hydrogen and $R^3$ denoting $C_1$–$C_4$-alkyl, or $R^2$ and $R^3$, together with the nitrogen atom to which they are bonded, being the piperazino, piperidino or morpholino radical, which are unsubstituted or are substituted by $C_1$–$C_4$-alkyl, $C_2$–$C_5$-carboalkoxy, pyridyl, naphthyl, phenyl or phenyl, which is substituted 1 to 3 times with halogen, hydroxyl, methyl, ethyl, methoxy, nitro or trifluoromethyl, and pharmaceutically tolerated salts thereof.

3. A process for the preparation of compounds of the formula I

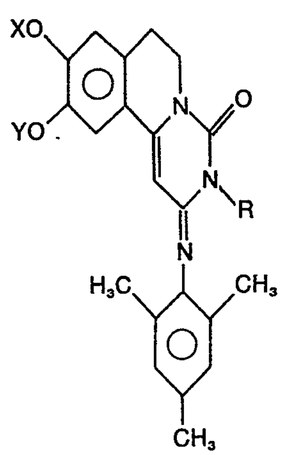

I

which comprises reacting a compound of the formula II

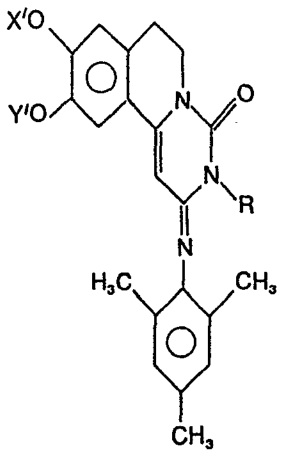

II

wherein one of the two substituents X′ and Y′ denotes hydrogen and the other denotes $C_1$–$C_4$-alkyl, with an epihalohydrin of the formula III

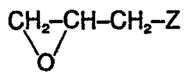

III

wherein Z denotes a halogen atom, and reacting the product obtained with a compound of the formula IV

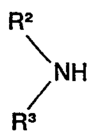

IV

wherein $R^2$ and $R^3$ have the abovementioned meanings.

4. A medicament containing a compound of the formula I mentioned in claim 1.

5. A compound of the formula I as defined in claim 1 or a pharmaceutically tolerated salt thereof for use as a medicament.

**Claims for the contracting State: AT**

1. A process for the preparation of a compound of the formula I

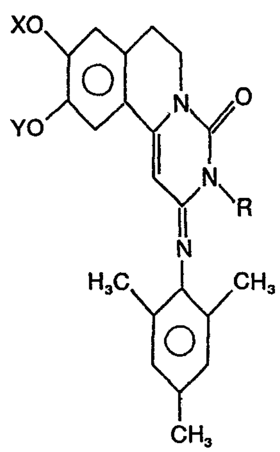

I

wherein R denotes $C_1$–$C_4$-alkyl, one of the two substituents X and Y is $C_1$–$C_4$-alkyl and the other is a radical of the formula $-CH_2CH(OR^1)CH_2NR^2R^3$ $R^1$, $R^2$ and $R^3$ being identical or different and denoting hydrogen or $C_1$–$C_4$-alkyl, or $R^2$ and $R^3$, together with the nitrogen atom to which they are bonded, denoting the piperazino, piperidino or morpholino radical, which are unsubstituted or are substituted by $C_1$–$C_4$-alkyl, $C_2$–$C_5$-carboalkoxy, pyridyl, naphthyl, phenyl or phenyl, which is 1 to 3 times substituted with halogen, hydroxy, methyl, ethyl, methoxy, nitro or trifluoromethyl, and a pharmaceutically tolerated salt thereof, which comprises reacting a compound of the formula II

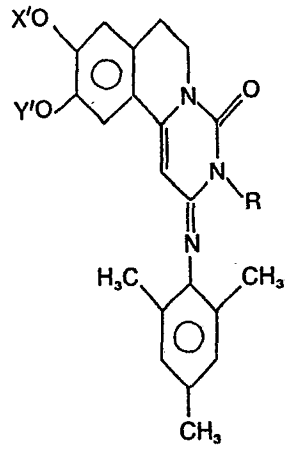

II

wherein one of the two substituents X′ and Y′ denotes hydrogen and the other denotes $C_1$–$C_4$-alkyl, with an epihalohydrin of the formula III

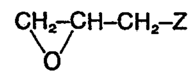

III

wherein Z denotes a halogen atom, and reacting the product obtained with a compound of the formula IV

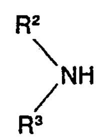

IV

wherein $R^2$ and $R^3$ have the abovementioned meanings.

2. A process as defined in claim 1, wherein a compound of the formula II, wherein R denotes methyl and one of the substituents X′ and Y′ is $C_1$–$C_4$-alkyl and the other is hydrogen, is at first reacted with a compound of the formula III, and the product obtained is reacted with a compound of the formula IV, wherein $R^2$ denotes hydrogen and $R^3$ denotes $C_1$–$C_4$-alkyl, or $R^2$ and $R^3$, together with the nitrogen atom to which they are bonded, being the piperazino, piperidino or morpholino radical, which are unsubstituted or are substituted by $C_1$–$C_4$-alkyl, $C_2$–$C_5$-carboalkoxy, pyridyl, naphthyl, phenyl or phenyl which is substituted 1 to 3 times with halogen, hydroxyl, methyl, ethyl, methoxy, nitro or trifluoromethyl.

**Revendications pour les Etats contractants: BE, CH, DE, FR, GB, IT, LI, NL, SE**

1. Composés répondant à la formule I

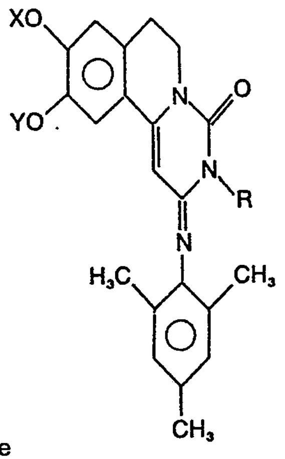

I

dans laquelle

R désigne un groupe alkyle en C1 à C4, un des deux substituants X et Y désigne un groupe alkyle en C1 à C4 et l'autre un radical répondant à la formule $-CH_2CH(OR^1)CH_2NR^2R^3$, dans laquelle $R^1$, $R^2$ et $R^3$ sont identiques ou différents et désignent l'hydrogène ou un groupe alkyle en C1 à C4, ou bien $R^2$ et $R^3$ forment avec l'atome d'azote auquel ils sont fixés, un radical pipérazino, morpholino ou pipéridino, qui peut être non substitué ou substitué par un groupe alkyle en C1 à C4, carbalcoxy en C2 à C5, pyridyle, naphtyle ou phényle, non substitué ou substitué 1 à 3 fois par un halogène, un groupe hydroxy, méthyle, éthyle, méthoxy, nitro ou trifluorométhyle, ainsi que leurs sels pharmaceutiquement acceptables.

2. Composés répondant à la formule I suivant la revendication 1, dans laquelle R désigne un groupe méthyle et un des substituants X et Y représente un groupe alkyle en C1 à C4 et l'autre un radical répondant à la formule $-CH_2CH(OR^1)CH_2NR^2R^3$ dans laquelle $R^1$ et $R^2$ désignent l'hydrogène et $R^3$ un groupe alkyle en C1 à C4, ou bien $R^2$ et $R^3$ forment avec l'atome d'azote auquel ils sont fixés, un radical pipérazino, morpholino ou pipéridino, qui est non substitué ou substitué par un groupe alkyle en C1 à C4, carbalcoxy en C2 à C5, pyridyle, naphtyle, phényle ou phényle substitué 1 à 3 fois par un halogène, un groupe hydroxy, méthyle, éthyle, méthoxy, nitro ou trifluorométhyle, ainsi que leurs sels pharmaceutiquement acceptables.

3. Procédés de préparation de composés répondant à la formule I

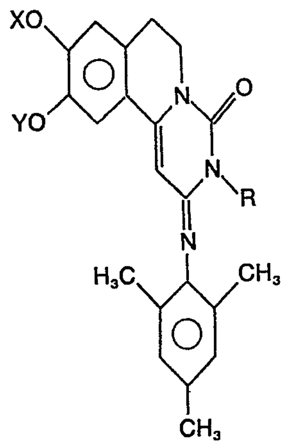

I

caractérisé en ce qu'on fait réagir un composé répondant à la formule II

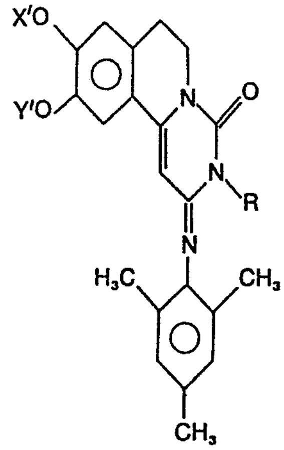

II

dans laquelle un des substituants X' et Y' désigne l'hydrogène et l'autre un groupe alkyle en C1 à C4, avec une épihalohydrine répondant à la formule III

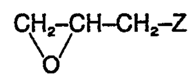

III

dans laquelle Z désigne un atome d'halogène, et on fait réagir le produit obtenu avec un composé répondant à la formule IV

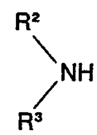

IV

dans laquelle $R^2$ et $R^3$ ont les significations indiquées ci-dessus.

4. Médicament, caractérisé en ce qu'il contient un composé répondant à la formule I donnée dans la revendication 1.

5. Composé répondant à la formule I suivant la revendication 1, ou un de ces sels pharmaceutiquement acceptables destiné à être utilisé comme médicament.

**Revendications pour l'Etat contractant: AT**

1. Procédé de préparation de composés répondant à la formule I

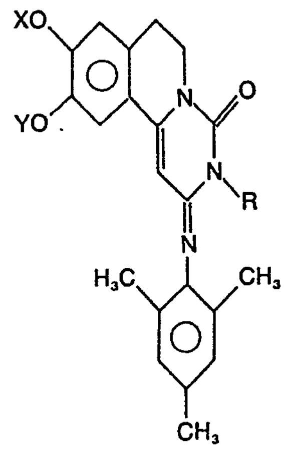

I

dans laquelle

R désigne un groupe alkyle en C1 à C4, un des deux substituants X et Y est un groupe alkyle en C1 à C4 et l'autre un radical répondant à la formule $-CH_2CH(OR^1)CH_2NR^2R^3$, dans laquelle $R^1$, $R^2$ et $R^3$ sont identiques ou différents et désignent l'hydrogène ou un groupe alkyle en C1 à C4, ou bien $R^2$ et $R^3$ forment avec l'atome d'azote auquel ils sont fixés, un radical pipérazino, morpholino ou pipéridino, qui peut être non substitué ou substitué par un groupe alkyle en C1 à C4, carbalcoxy en C2 à C5, pyridyle, naphtyle, phényle ou phényle substitué 1 à 3 fois par un halogène, un groupe hydroxy, méthyle, éthyle, méthoxy, nitro ou trifluorométhyle, ainsi que leurs sels pharmaceutiquement acceptables, caractérisé en ce qu'on fait réagir un composé répondant à la formule II

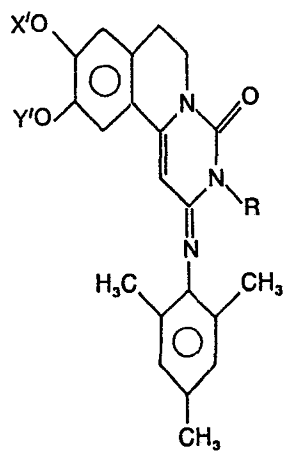

II

dans laquelle un des deux substituants X' et Y' est l'hydrogène et l'autre un groupe alkyle en C1 à C4, avec une épihalohydrine répondant à la formule III

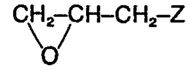

III

dans laquelle Z désigne un atome d'halogène, et on fait réagir le produit obtenu avec un composé répondant à la formule IV

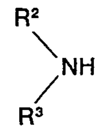

IV

dans laquelle $R^2$ et $R^3$ ont les significations indiquées ci-dessus, et on transforme le cas échéant le composé répondant à la formule I obtenu en un sel pharmaceutiquement acceptable.

2. Procédé suivant la revendication 1, caractérisé en ce qu'on fait réagir un composé répondant à la formule II, dans laquelle R désigne un groupe méthyle, et un des substituants X' et Y' représente un groupe alkyle en C1 à C4 et l'autre un atome d'hydrogène, tout d'abord avec un composé de formule III, puis on fait réagir le composé obtenu avec un composé de formule IV dans laquelle $R^2$ représente l'hydrogène et $R^3$ un groupe alkyle en C1 à C4, ou bien $R^2$ et $R^3$ forment avec l'atome d'azote auquel ils sont fixés un radical pipérazino, pipéridino ou morpholino, qui peut être non substitué ou substitué par un groupe alkyle en C1 à C4, carbalcoxy en C2 à C5, pyridyle, naphtyle, phényle ou phényle substitué 1 à 3 fois avec un halogène, un groupe hydroxy, méthyle, éthyle, méthoxy, nitro ou trifluorométhyle.